# EUROPEAN PATENT SPECIFICATION

(11) **EP 4 202 036 B1**
(45) Date of publication and mention of the grant of the patent: **09.09.2026**
(21) Application number: 22206407.3
(22) Date of filing: 19.07.2016
(51) Int. Cl.: C12N 5/02, C12N 5/0789, C12N 5/0797, A61K 35/28, A61K 35/44, A61P 7/00, A61P 7/06, A61P 31/04, A61P 31/18, A61P 37/00, A61P 37/04, A61P 43/00

(54) **METHODS AND COMPOSITIONS FOR STEM CELL TRANSPLANTATION**
VERFAHREN UND ZUSAMMENSETZUNGEN ZUR STAMMZELLTRANSPLANTATION
PROCÉDÉS ET COMPOSITIONS POUR LA TRANSPLANTATION DE CELLULES SOUCHES

(30) Priority: 20.07.2015 US 201562194460 P
(43) Date of publication of application: 28.06.2023
(62) Document number(s) of the earlier application(s) in accordance with Art. 76 EPC: 16828392.7 / 3 325 612
(73) Proprietor: Angiocrine Bioscience, Inc., San Diego, CA 92121 (US)
(72) Inventor: FINNEGAN, Paul, William, Del Mar, CA 92014 (US); DAVIS, Claude, Geoffrey, Auburn, CA 95602 (US); GINSBERG, Michael, Daniel, San Diego, CA 92121 (US); NOLAN, Daniel, Joseph, Hawthorne, NY 10532 (US)
(74) Representative: Barker Brettell LLP

(56) References cited:
- WO-A1-95/11692
- RAYNAUD CHRISTOPHE M ET AL: "Endothelial cells provide a niche for placental hematopoietic stem/progenitor cell expansion through broad transcriptomic modification", STEM CELL RESEARCH, vol. 11, no. 3, 9 August 2013 (2013-08-09), pages 1074 - 1090, XP028761063, ISSN: 1873-5061, DOI: 10.1016/J.SCR.2013.07.010
- JASON M. BUTLER ET AL: "Endothelial Cells Are Essential for the Self-Renewal and Repopulation of Notch-Dependent Hematopoietic Stem Cells", CELL STEM CELL, vol. 6, no. 3, 1 March 2010 (2010-03-01), AMSTERDAM, NL, pages 251 - 264, XP055267442, ISSN: 1934-5909, DOI: 10.1016/j.stem.2010.02.001
- HIDEKI KOBAYASHI ET AL: "Angiocrine factors from Akt-activated endothelial cells balance self-renewal and differentiation of haematopoietic stem cells", NATURE CELL BIOLOGY, vol. 12, no. 11, 24 October 2010 (2010-10-24), pages 1046 - 1056, XP055174385, ISSN: 1465-7392, DOI: 10.1038/ncb2108
- LI HUILIN ET AL: "Arterial endothelium creates a permissive niche for expansion of human cord blood hematopoietic stem and progenitor cells", vol. 11, no. 1, 14 August 2020 (2020-08-14), XP093036197, Retrieved from the Internet <URL:https://link.springer.com/article/10.1186/s13287-020-01880-8/fulltext.html> DOI: 10.1186/s13287-020-01880-8
- BENJAMIN BOYERINAS ET AL: "HSPCs in the Balance: The Vascular Niche", CELL STEM CELL, vol. 7, no. 6, 3 December 2010 (2010-12-03), pages 645 - 646, XP028184574, ISSN: 1934-5909, [retrieved on 20101122], DOI: 10.1016/J.STEM.2010.11.021
- M. SEANDEL ET AL: "Generation of a functional and durable vascular niche by the adenoviral E4ORF1 gene", PROCEEDINGS OF THE NATIONAL ACADEMY OF SCIENCES, vol. 105, no. 49, 9 December 2008 (2008-12-09), pages 19288 - 19293, XP055495348, ISSN: 0027-8424, DOI: 10.1073/pnas.0805980105

## Description

### CROSS-REFERENCE TO RELATED APPLICATIONS

This application claims the benefit of priority of U.S. Provisional Patent Application No. 62/194,460 filed on July 20, 2015,

### BACKGROUND

### Field

The present teachings relate to methods and compositions for improved stem cell therapy, and in particular, to methods and compositions for facilitating the expansion and engraftment of hematopoietic stem and progenitor cells and immune reconstitution of the hematopoietic system of a stem cell transplant recipient.

### Introduction

Hematopoietic stem or progenitor cell transplantation (HSCT) is a critical component of the treatment of a wide array of hematologic disorders. Generally, there are two types of HSCTs: autologous and allogeneic transplantation. Autologous transplantation involves infusion of a recipient's own cells following myeloablative treatment. Autologous cell transplants minimize the risk of graft versus host disease (GVHD) and result in reduced complications. Allogeneic transplantation involves infusion of donor stem cells, typically using a donor that matches the recipient's MHC. However, matched unrelated donor (MUD) transplants are also associated with a stronger graft versus host reaction, and thus result in higher mortality rates.

There are three main sources of hematopoietic stem and/or progenitor cells (HSPCs): bone marrow, peripheral blood, and umbilical cord blood. Umbilical cord blood (UCB) is a practical alternative source to other hematopoietic progenitor sources (*e.g*., bone marrow and mobilized peripheral blood) for related and unrelated allogeneic hematopoietic stem cell transplantation. Unfortunately, however, although cord blood is readily available and shows lower incidences of graft versus host disease, it is characterized by delayed engraftment of the multilineage cells (*e.g*., neutrophils, platelets, erythrocytes), all of which are important for a successful and clinically meaningful immune reconstitution. Accordingly, while there is enormous promise for treating hematologic disorders with HSPCs obtained from cord blood, the slow rate of hematopoietic recovery remains a major obstacle, Laughlin, et al., N. Eng. J. Med. 351:22; 2265-2275 (2004), and the efficiency of engraftment is still significantly less than with HSC from bone marrow or peripheral blood. Rocha et al., N. Eng. J. Med. 351:22; 2276-2285 (2004).

Hematopoietic stem cells can be expanded in distinct media compositions comprising a mixture of cytokines. Stem cell expansion techniques are known in the art, including, U.S. Pat. No. 6,326,198, U.S. Pat. No. 6,338,942; U.S. Pat. No. 6,335,195. Kobayashi *et al.* and Butler *et al.* recently described the generation of a pro-hematopoietic vascular niche generated from primary human endothelial cells (EC) by transduction with the adenoviral E4ORF1 gene, which minimally activates the Akt/Mtor pathway leading to expression of pro-hematopoietic survival and proliferation signals. Kobayashi et al., Nature Cell Biology 12, 1046-1056 (2010); Butler et al., Blood 120, 1344-1347 (2012). Typical HSPC/EC expansion procedures involve co-culturing HSPCs on a monolayer of ECs deposited in a small cell-culture flask, using initial EC:HSPC ratios from 6:1 to 3:1. After 24-48 hours the HSPCs are removed and deposited on a further monolayer of ECs in second flask, and so on until the required number of HSPCs is obtained. One significant draw-back to such flask-based expansions, however, is the high number of open events, as many as 1,800 for a typical procedure, which dramatically increase the risk of contamination.

The need for *in vitro* culture methods and systems capable of clinically useful amplification of human CD34⁺ HSPCs as well as other hematopoietic elements continues. A system capable of producing large quantities of certain bone marrow elements is needed to supply sufficient quantities of these elements for use in human therapeutics, for example, as bone marrow transplantation, transfusable blood components or genetic therapy. The methods described herein meet these and other needs.

### SUMMARY OF THE INVENTION

The present invention addresses and successfully resolves this long-standing need in the art, providing unprecedented levels of HSPC expansion by co-culturing HSPC with endothelial cells in closed-system bioreactors using initial EC/HSPC ratios of at least 25:1, 50:1, 100:1, or 500:1, and still more preferably as high as 1000:1, 2000:1 or 3000:1. Significantly, the interaction between the HSPCs and the ECs remains undisturbed during the expansion step, thereby maintaining continuous intercellular communication between the HSPCs and the ECs, enhancing the efficiency and overall output of the expansion. Furthermore, the number of open events is reduced compared to open cell culture environments, such as culture flasks, thereby greatly reducing the risk of contamination.

The HSPCs expanded according to the methods described herein have beneficial pharmacological effects, particularly when administered to an HSCT recipient in combination with the ECs, wherein a continuous interaction is maintained between the HSPCs and the ECs throughout expansion and administration. The transient existence of these allogeneic endothelial cells in blood circulation and/or in tissue offer beneficial *in vivo* outcomes in HSCT such as enhanced multi-lineage engraftment and greater duration of engraftment, as well as relatively rapid initial engraftment, as measured, for example, by neutrophil counts. As demonstrated for the first time herein, this is true even if the endothelial cells are irradiated prior to co-culturing with HSCs and cannot replicate.

Accordingly, one aspect of the present disclosure relates to methods of stem cell transplantation in a subject in need thereof comprising: (a) expanding hematopoietic stem or progenitor cells (HSPCs) by contacting the HSPCs with endothelial cells (ECs) at an initial EC/HSPC ratio for a first time period under conditions allowing for expansion of the HSPCs, to produce an expanded cell population comprising HSPCs and ECs at an expanded HSPC/EC ratio; and (b) transfusing the expanded cell population obtained from step (a) into the subject;
wherein a continuous interaction is maintained between the HSPCs and the ECs throughout the expansion and the transfusion steps.

A further aspect of the present invention relates to compositions comprising HSPCs and ECs for use in stem cell transplantation in a subject in need thereof, wherein the stem cell transplantation comprises the methods described herein. Preferably, the ECS are in continuous proximity with the HSPCs throughout the expansion and transfusion steps, as described and shown herein. Still more preferably, the ECs are in continuous contact with the HSPCs during at least the expansion step.

In some embodiments, the initial EC/HSPC ratio is at least about 200:1. In some embodiments, the initial EC/HSPC ratio is at least about 300:1. In some embodiments, the initial EC/HSPC ratio is at least about 400:1. In some embodiments, the initial EC/HSPC ratio is at least about 500:1. In some embodiments, the initial EC/HSPC ratio is at least about 600:1. In some embodiments, the initial EC/HSPC ratio is at least about 700:1. In some embodiments, the initial EC/HSPC ratio is at least about 1000:1. In some embodiments, the initial EC/HSPC ratio is at least about 2000:1. In some embodiments, the initial EC/HSPC ratio is at least about 3000:1.

In some embodiments, the expanded HSPC/EC ratio is about 10:1 to about 1:10. In some embodiments, the expanded HSPC/EC ratio is about 10:1 to about 1:1. In some embodiments, the expanded HSPC/EC ratio is about 9:1 to about 2:1. In some embodiments, the expanded HSPC/EC ratio is about 8:1 to about 3:1. In some embodiments, the expanded HSPC/EC ratio is about 7:1 to about 4:1. In some embodiments, the expanded HSPC/EC ratio is about 6:1 to about 5:1.

Disclosed but not part of the invention are kits or systems for isolating, expanding, preserving and administering HSPCs and endothelial cells in accordance with the methods described herein.

### BRIEF DESCRIPTION OF THE DRAWINGS

The skilled artisan will understand that the drawings are for illustration purposes only, and are not intended to limit the scope of the present teachings in any way.

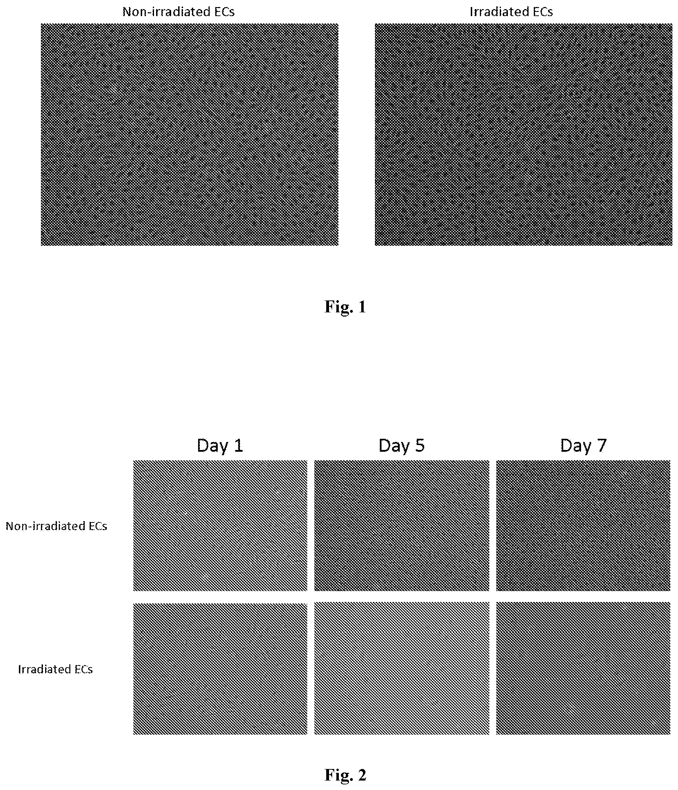

**Figure 1** shows phase microscopy images of the effect of radiation exposure on E4ORF1+ engineered ECs. ECs were harvested under standard culture conditions and transduced with E40RF1. The cells were then split into two flasks and further cultured until 100% confluent. One vessel was left un-manipulated (left panel) and the other vessel was irradiated with 1500cGy from a cesium irradiator (right panel). No differences were detectable between the two flasks by phase microscopy.
**Figure 2** shows serial phase microscopy images of irradiated (bottom row) and non-irradiated (top row) E4ORF1+ engineered ECs over the period of one week in culture. Two flasks of cells were cultured to semi-confluence, at which point one flask (bottom panels) was irradiated with 1500cGy using a cesium irradiator. Both flasks were further cultured for one week and phase microscopy images were generated on days 1, 5, and 7 post-irradiation.

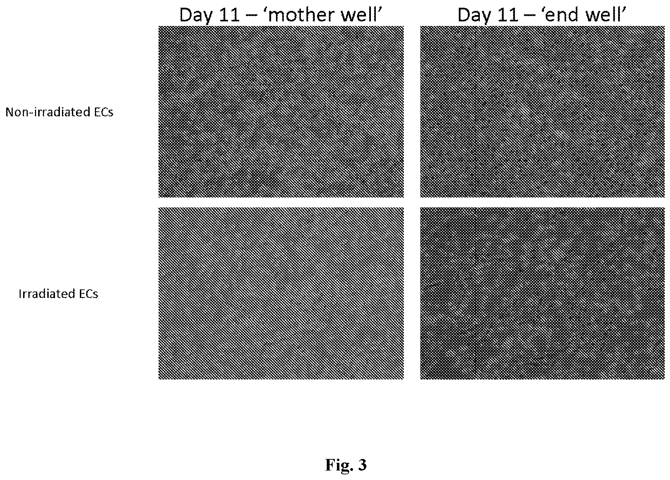

**Figure 3** shows phase microscopy images of HSPCs in co-culture with irradiated (bottom row) and non-irradiated (top row) E4ORF1+ engineered ECs. Purified HSPCs (CD34+) from umbilical cord blood were co-cultured in 6-well plates with confluent monolayers of non-irradiated and 1500cGy irradiated E4ORF1 ECs. At the conclusion of the 11 day expansion and serial passaging of the cells, phase microscopy images were generated of both the original culture well ("mother well," left column) and one of the final wells ("end well," right column) generated by serial passaging.

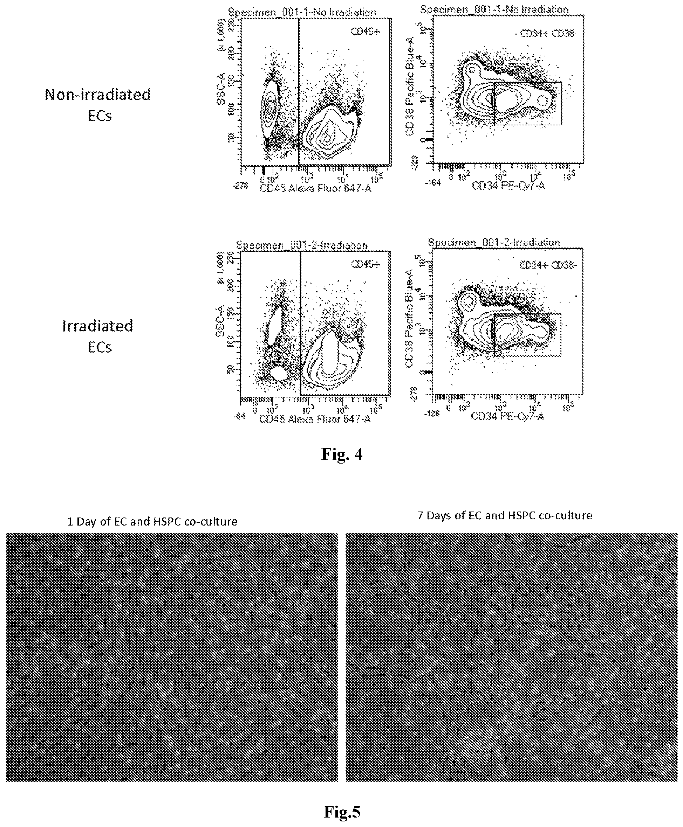

**Figure 4** shows flow cytometry data of co-cultures of HSPCs with irradiated (bottom panels) and non-irradiated E4ORF1+ engineered ECs (top panels). Purified HSPCs (CD34+) from umbilical cord blood were co-cultured in 6-well plates with confluent monolayers of either irradiated or non-irradiated E4ORF1 ECs. At the conclusion of the 11 day expansion, the hematopoietic cells were analyzed by flow cytometry. The left column shows results of cells stained for the expression of CD45 (a pan hematopoietic cell surface marker). The right panel shows results of cells doubly stained for expression of CD34 (a positive marker for HSPCs) and CD38 (a negative marker for HSPCs).
**Figure 5** shows representative phase microscopy images of short (left panel) and long (right panel) term HSPC-E4ORF1+ engineered EC co-culture and the resulting EC attrition. Purified HSPCs (CD34+) from umbilical cord blood were co-cultured in a 6-well plate with confluent monolayers of ECs. As the population of HSPCs expands, the non-adherent cells are gently removed and placed on a larger and fresh layer of ECs. The original well, termed "mother well" is refed with additional media. This process is repeated approximately every 48 hours to all co-cultured wells. During the 7 days of co-culture, the E4ORF1+ engineered ECs in the mother well are depleted (right panel) compared to only 1 day of co-culture of the same expanding HSPCs plated on fresh E4ORF1 ECs (left panel).

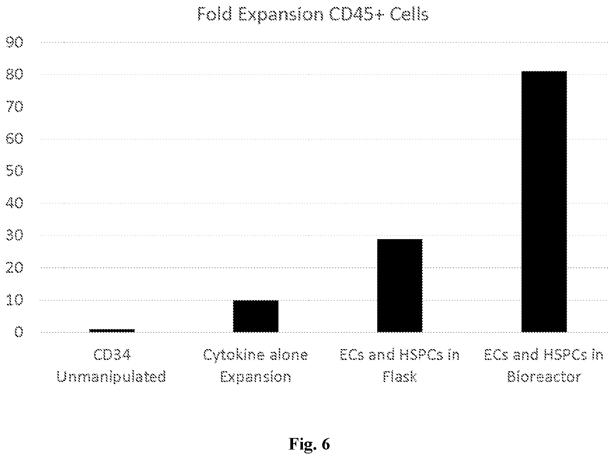

**Figure 6** shows the quantification of total hematopoietic cell expansion in a bioreactor with continuous E4ORF1+ engineered EC and HSPC contact compared to expansion with cytokines alone and to expansion with discontinuous E4ORF1+ engineered EC and HSPC contact. Purified HSPCs (CD34+) from umbilical cord blood were expanded in a flask with cytokines alone, in a flask on monolayers of E4ORF1+ engineered ECs, or in a hollow fiber bioreactor preloaded with E4ORF1+ engineered ECs. The cytokine alone expansion and the expansion on E4ORF1+ engineered ECs in a flask required a disruption to the culture process to rinse and replate the cells during the expansion. Cell counting and flow cytometric enumeration of the CD45+ cell population was performed on day 6 of culture.

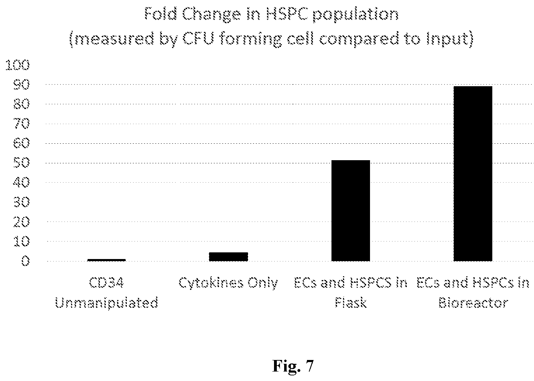

**Figure 7** represents quantification of colony forming assays of HSPC expansion in a bioreactor with continuous E4ORF1+ engineered EC and HSPC contact compared to expansion with cytokines alone and expansion with discontinuous E4ORF1+ engineered EC and HSPC contact. The abbreviation "CFU" refers to colony-forming units. The frequencies of colonies in multiple microscope fields of view were used to extrapolate the number of CFUs in the total expanded population.

### DETAILED DESCRIPTION

### Definitions

In reference to the present disclosure, the technical and scientific terms used in the descriptions herein will have the meanings commonly understood by one of ordinary skill in the art, unless specifically defined otherwise. Accordingly, the following terms are intended to have the following meanings:
"Allogeneic" refers to deriving from, originating in, or being members of the same species, where the members are genetically related or genetically unrelated but genetically similar. An "allogeneic transplant" refers to transfer of cells or organs from a donor to a recipient, where the recipient is the same species as the donor.

"Autologous" refers to deriving from or originating in the same subject or patient. An "autologous transplant" refers to collection and re-transplant of a subject's own cells or organs.

"Committed myeloid progenitor cell" or "myeloid progenitor cell" or "MP" refers to a multipotent or unipotent progenitor cell capable of ultimately developing into any of the terminally differentiated cells of the myeloid lineage, but which do not typically differentiate into cells of the lymphoid lineage. Hence, "myeloid progenitor cell" refers to any progenitor cell in the myeloid lineage. Committed progenitor cells of the myeloid lineage include oligopotent CMP, GMP, and MEP as defined herein, but also encompass unipotent erythroid progenitor, megakaryocyte progenitor, granulocyte progenitor, and macrophage progenitor cells. Different cell populations of myeloid progenitor cells are distinguishable from other cells by their differentiation potential, and the presence of a characteristic set of cell markers.

"Common myeloid progenitor cell" or "CMP" refers to a cell characterized by its capacity to give rise to granulocyte/monocyte (GMP) progenitor cells and megakaryocyte/ erythroid (MEP) progenitor cells. These progenitor cells have limited or no self-renewing capacity, but are capable of giving rise to myeloid dendritic, myeloid erythroid, erythroid, megakaryocytes, granulocyte/macrophage, granulocyte, and macrophage cells.

"Continuous interaction" as used herein refers to at least the continuous proximity of the HSPCs to the ECs during one or more process steps described herein. "Proximity" in this context refers to the combined presence of the HSPCs and the ECs in the same medium, for example a cell culture medium, a transfusion medium, or in any medium used during any optional step between the expansion and transfusion steps, such as harvesting the expanded cell population, or preparing a pharmaceutical composition comprising the expanded cell population. In some embodiments, the HSPCs and the ECs are in continuous proximity during the expansion and transfusion steps combined. In other words, the HSPCs and the ECs are simultaneously present in the same medium throughout the expansion step and until the transfusion step is complete. For example, at no point during the expansion step are the HSPCs removed from the expansion medium, *e.g.*, to contact the HSPCs with additional ECs. Furthermore, in some embodiments, the methods described herein do not comprise any purification step whereby the endothelial cells are removed from the expanded cell population comprising the expanded HSPCs prior to the transfusion step. "Continuous interaction" as used herein may also refer to the continuous contact between the HSPCs the ECs during one or more process steps described herein. In some embodiments, the HSPCs and the ECs are in continuous contact during the expansion step. In this context, the term "contact" between the HSPCs and the endothelial cells refers to a spatial relationship wherein the distance between the HSPCs and the ECs is ≤ 500 µm. In some embodiments, the distance between the HSPCs and the ECs is ≤ 400 µm. In some embodiments, the distance between the HSPCs and the ECs is ≤ 300 µm. In some embodiments, the distance between the HSPCs and the ECs is ≤ 200 µm. In some embodiments the distance between the HSPCs and the ECs is less than or equal to an internal dimension of the vessel in which the expansion is performed.

"Culturing" refers to the propagation of cells or organisms on or in media of various kinds. "Co-culturing" refers to the propagation of two or more distinct types of cells or organisms on or in media of various kinds, for instance, in some embodiments, HSPCs and endothelial cells may be co-cultured.

The term "engineered" when used in relation to cells herein refers to cells that have been engineered by man to result in the recited phenotype (e.g. E4ORF1+), or to express a recited nucleic acid molecule or polypeptide. The term "engineered cells" is not intended to encompass naturally occurring cells, but is, instead, intended to encompass, for example, cells that comprise a recombinant nucleic acid molecule, or cells that have otherwise been altered artificially (e.g. by genetic modification), for example so that they express a polypeptide that they would not otherwise express, or so that they express a polypeptide at substantially higher levels than that observed in non-engineered endothelial cells.

"Expansion" in the context of cells refers to increase in the number of a characteristic cell type, or cell types, from an initial population of cells, which may or may not be identical. The initial cells used for expansion need not be the same as the cells generated from expansion. For instance, the expanded cells may be produced by growth and differentiation of the initial population of cells. Excluded from the term expansion are limiting dilution assays used to characterize the differentiation potential of cells.

"Genetic modification" or "gene-modified" refers to any addition, deletion or disruption to a cell's normal nucleotides. The methods of this invention are intended to encompass any genetic modification method of exogenous or foreign gene transfer (or nucleic acid sequence transfer) into HSPCs or endothelial cells. The term "genetic modification" encompasses use of a gene delivery vehicle and includes but is not limited to transduction (viral mediated transfer of nucleic acid to a recipient, either *in vivo* or *in vitro*), transfection (uptake by cells of isolated nucleic acid), liposome mediated transfer and others means well known in the art.

"Graft-versus-host disease" or "GVH" or "GVHD" refers to a cellular response that occurs when lymphocytes of a different MHC class are introduced into a host, resulting in the reaction of the lymphocytes against the host.

"Granulocyte/macrophage progenitor cell" or "GMP" refers to a cell derived from common myeloid progenitor cells, and characterized by its capacity to give rise to granulocyte and macrophage cells, but which does not typically give rise to erythroid cells or megakaryocytes of the myeloid lineage.

"Hematopoietic disease" refers to any blood disorder including but not limited to hematopoietic malignancy, hemoglobinopathy, and immunodeficiency.

"Hematopoietic stem or progenitor cell" or "HSPC" encompasses HSCs, as defined herein, as well as multipotent non-self-renewing progenitor cells that are capable of ultimately differentiating into all cell types of the hematopoietic system, and oligopotent and unipotent progenitor cells capable differentiating into certain cell types of the hematopoietic system. HSPCs include CMPs, MPs, MEPs, and GMPs, as defined herein.

"Hematopoietic stem cell" or "HSC" refers to clonogenic, self-renewing pluripotent cell capable of ultimately differentiating into all cell types of the hematopoietic system, including B cells T cells, NK cells, lymphoid dendritic cells, myeloid dendritic cells, granulocytes, macrophages, megakaryocytes, and erythroid cells. As with other cells of the hematopoietic system, HSCs are typically defined by the presence of a characteristic set of cell markers.

"Isolated" refers to a product, compound, or composition which is separated from at least one other product, compound, or composition with which it is associated in its naturally occurring state, whether in nature or as made synthetically.

"Marker phenotyping" refers to identification of markers or antigens on cells for determining its phenotype (*e.g*., differentiation state and/or cell type). This may be done by immunophenotyping, which uses antibodies that recognize antigens present on a cell. The antibodies may be monoclonal or polyclonal, but are generally chosen to have minimal crossreactivity with other cell markers. It is to be understood that certain cell differentiation or cell surface markers are unique to the animal species from which the cells are derived, while other cell markers will be common between species. These markers defining equivalent cell types between species are given the same marker identification even though there are species differences in structure (*e.g*., amino acid sequence). Cell markers include cell surfaces molecules, also referred to in certain situations as cell differentiation (CD) markers, and gene expression markers. The gene expression markers are those sets of expressed genes indicative of the cell type or differentiation state. In part, the gene expression profile will reflect the cell surface markers, although they may include non-cell surface molecules.

"Megakaryocyte/erythroid progenitor cell" or "MEP" refers to a cell derived from common myeloid progenitor cells, and characterized by its capacity to gives rise to erythroid cells and megakaryocytes, but which does not typically give rise to granulocytes, macrophages, or myeloid dendritic cells.

"Myeloablative" or "myeloablation" refers to impairment or destruction of the hematopoietic system, typically by exposure to a cytotoxic agent or radiation. Myeloablation encompasses complete myeloablation brought on by high doses of cytotoxic agent or total body irradiation that destroys the hematopoietic system. It also includes a less than complete myeloablated state caused by non-myeloablative conditioning. Thus, a non-myeloablative conditioning is treatment that does not completely destroy the subject's hematopoietic system.

"Self-renewal" refers to the ability of a cell to divide and generate at least one daughter cell with the identical (e.g., self-renewing) characteristics of the parent cell. The second daughter cell may commit to a particular differentiation pathway. For example, a self-renewing hematopoietic stem cell divides and forms one daughter stem cell and another daughter cell committed to differentiation in the myeloid or lymphoid pathway. A committed progenitor cell has typically lost the self-renewal capacity, and upon cell division produces two daughter cells that display a more differentiated (i.e., restricted) phenotype.

"Sorting" as it pertains to cells refers to separation of cells based on physical characteristics or presence of markers (such as sorting using side scatter (SSC) and forward scatter (FSC), or fluorescence activation cell sorting (FACS) using labeled antibodies), or analysis of cells based on presence of cell markers, e.g., FACS without sorting.

"Stem cell" refers to stem cells of various cell types, such as, muscle, epithelial, neural and bone stem cells. More particularly the invention is concerned with HSPCs.

"Subject" or "patient" are used interchangeably and refer to, except where indicated, mammals such as humans and non-human primates, as well as rabbits, rats, mice, goats, pigs, and other mammalian species.

"Substantially pure cell population" refers to a population of cells having a specified cell marker characteristic and differentiation potential that is at least about 50%, preferably at least about 75-80%, more preferably at least about 85-90%, and most preferably at least about 95% of the cells making up the total cell population. Thus, a "substantially pure cell population" refers to a population of cells that contain fewer than about 50%, preferably fewer than about 20-25%, more preferably fewer than about 10-15%, and most preferably fewer than about 5% of cells that do not display a specified marker characteristic and differentiation potential under designated assay conditions.

"Therapeutic gene" refers to an entire gene or only the functionally active fragment of the gene. Therapeutic gene may be capable of compensating for a deficiency in a patient that arises from a defective or absent endogenous gene. Additionally, a therapeutic gene may be one that antagonizes production or function of an infectious agent, antagonizes pathological processes, improves a host's genetic makeup, facilitates engraftment, or a stem cell's therapeutic potency.

"Xenogeneic" refers to deriving from, originating in, or being members of different species, *e.g.,* human and rodent, human and swine, human and chimpanzee, *etc.* A "xenogeneic transplant" refers to transfer of cells or organs from a donor to a recipient where the recipient is a species different from that of the donor.

### Expansion of Hematopoietic Stem or Progenitor Cells

The present disclosure describes methods, compositions, and systems or kits for facilitating HSPC engraftment. In one aspect, methods are provided for enhancing engraftment of HSPCs in a patient in need thereof, which comprises co-culturing and co-infusing HSPCs with ECs while maintaining a continuous interaction between the HSPCs and the ECs throughout. As demonstrated for the first time herein, maintaining a continuous interaction between the HSPCs and the ECs throughout the expansion and transfusion steps increases the efficiency of the expansion and the engraftment of the resulting expanded HSPCs, thereby improving the speed and completeness of immune reconstitution, and hence the survival of transplant patients receiving an HSPC graft.

Hematopoietic stem cells are pluripotent stem cells capable of self-renewal and are characterized by their ability to give rise under permissive conditions to all cell types of the hematopoietic system. HSC self-renewal refers to the ability of an HSC cell to divide and produce at least one daughter cell with the same self-renewal and differentiation potential of an HSC; that is, cell division gives rise to additional HSCs. Self-renewal provides a continual source of undifferentiated stem cells for replenishment of the hematopoietic system. The marker phenotypes useful for identifying HSCs will be those commonly known in the art. For human HSCs, the cell marker phenotypes preferably include CD34⁺ CD38⁻ CD90(Thy1)⁺ Lin⁻. For mouse HSCs, an exemplary cell marker phenotype is Sca-1⁺ CD90⁺ (see, *e.g.*, Spangrude, G. J. et al., Science 1:661-673 (1988)) or c-kit⁺ Thy^{lo} Lin⁻ Sca-1⁺ (see, Uchida, N. et al., J. Clin. Invest. 101(5):961-966 (1998)). Alternative HSC markers such as aldehyde dehydrogenase (see Storms et al., Proc. Nat'l Acad. Sci. 96:9118-23 (1999), AC133 (see Yin et al., Blood 90:5002-12 (1997), and CD150 (SLAM) (see Kiel Cell 2005, 121(7) 1109-21) may also find advantageous use.

HSCs can be obtained from a variety of sources, including bone marrow, peripheral blood, cord blood, and other sources known to harbor hematopoietic and myeloid progenitor cells, including liver, particularly fetal liver. Peripheral and cord blood is a rich source of HSCs. Cells are obtained using methods known and commonly practiced in the art. For example, methods for preparing bone marrow cells are described in Sutherland et al., Bone Marrow Processing and Purging: A Practical Guide (Gee, A. P. ed.), CRC Press Inc. (1991)). HSCs can also be derived from primordial stem cell sources such as embryonic stem cells and germ cells (Shamblott et al., Proc. Natl. Acad. Sci. USA 95:13726 (1998)) using appropriate expansion and differentiation techniques.

HSC cells are derived from any animal species with a hematopoietic system, as generally described herein. Preferably, suitable animals will be mammals, including, by way of example and not limitation, rodents, rabbits, canines, felines, pigs, horses, cows, primates (*e.g*., human), and the like.

One aspect of the present invention relates to methods of stem cell transplantation in a subject in need thereof comprising: (a) expanding hematopoietic stem or progenitor cells (HSPCs) by contacting the HSPCs with endothelial cells (ECs) at an initial EC/HSPC ratio for a first time period under conditions allowing for expansion of the HSPCs, to produce an expanded cell population comprising HSPCs and ECs at an expanded HSPC/EC ratio; and (b) transfusing the expanded cell population obtained from step (a) into the subject; wherein a continuous interaction is maintained between the HSPCs and the ECs throughout the expansion and the transfusion steps..

A further aspect of the present invention relates to compositions comprising HSPCs and ECs for use in stem cell transplantation in a subject in need thereof, wherein the stem cell transplantation comprises: (a) expanding HSPCs by contacting the HSPCs with ECs at an initial EC/HSPC ratio for a first time period under conditions allowing for expansion of the HSPCs, to produce an expanded cell population comprising HSPCs and ECs at an expanded HSPC/EC ratio; and (b) transfusing the expanded cell population obtained from step (a) into the subject; wherein a continuous interaction is maintained between the HSPCs and the ECs throughout the expansion and the transfusion steps.

In some embodiments, the HSPCs are hematopoietic stem cells. In some embodiments, the HSPCs are allogeneic with respect to the subject. In some embodiments, the HSPCs are umbilical cord blood HSPCs. In some embodiments, the HSPCs are autologous with respect to the subject. In some embodiments, the HSPCs are gene-modified steady state bone marrow derived HSPCs.

In some preferred embodiments the endothelial cells are genetically modified. The adenoviral early 4 (E4) region contains at least 6 open reading frames (E4ORFs). The entire E4 region has been shown to regulate angiogenesis and promote survival, but not proliferation, of endothelial cells (see, for example, Zhang et al. (2004), J. Biol. Chem. 279(12):11760-66). Use of the entire E4 region, either clinically or experimentally, to induce angiogenesis or to promote survival or proliferation of endothelial cells, may not be desirable because some of the E4ORFs can have deleterious effects. For example, the E4ORF6 gene is known to induce apoptosis (Yamano et al. (1999) J. Virol. 73:10095-103). Also, the E4ORFs are immunogenic and therefore administration of all of the E4ORFs to subjects may not be desirable. Rafii et al., (WO2008089448) identified sequences within the E4ORF region that are useful for inducing angiogenesis and for promoting survival and proliferation of endothelial cells.

Several members of the E-twenty six (ETS)-family of transcription factors (TFs), including ETV2 (Lee et al., Cell stem cell, 2: 497-507 (2008); Sumanas et al., Blood, 111: 4500-4510 (2008)), FLU (Liu et al., Current Bio. 18: 1234-1240 (2008)), and ERG (McLaughlin et al., Blood, 98: 3332-3339 (2001)) have been implicated in regulating vascular development and angiogenesis (De Val et al., Dev Cell, 16: 180-195 (2009); Sato et al., Cell Struct Funct, 26: 19-24 (2001)). These TFs directly regulate the expression of genes associated with EC development and function. Adult ECs constitutively express several ETS factors, such as FLU, ERG (isoforms 1 and 2), ETS1, ETS2, Elfl, Elkl, VEZF and ETV6, while ETV2 is transiently expressed during embryonic development and is absent in adult ECs (Kataoka et al., Blood, 118: 6975-6986 (2011); Lelievre et al., The International Journal Of Biochemistry and Cell Biology, 33: 391-407 (2001)).

In some embodiments, the ECs are engineered to express an adenovirus E4ORF1 polypeptide (i.e. they are E4ORF1+ engineered endothelial cells). In some embodiments, the endothelial cells are engineered to express one or more ETS family transcription factors, such as those listed above (i.e. they are ETS+ engineered endothelial cells). In some embodiments, the endothelial cells are engineered to express the ETS family transcription factor ETV2 (i.e. they are ETV2+ engineered endothelial cells). In some embodiments, the ECs are E4ORF1+ ETV2+ engineered endothelial cells. In some embodiments, the ECs are E4ORF1+ ETS+ engineered endothelial cells. In some embodiments, the ECs are also E4ORF6+. In some embodiments, the ECs comprise a recombinant nucleic acid molecule that encodes an ETS family transcription factor. In some embodiments, the ECs comprise a recombinant nucleic acid molecule that encodes an ETV2 polypeptide. In some embodiments, the ECs comprise a recombinant nucleic acid molecule that encodes an adenovirus E4ORF1 polypeptide. In some embodiments, ECs comprise a recombinant nucleic acid molecule that encodes an adenovirus E4ORF6 polypeptide. In some embodiments, the nucleic acid molecule is in the form of a plasmid vector. In some embodiments, the nucleic acid molecule is integrated into the genomic DNA of the engineered ECs. In some embodiments, the ECs are differentiated ECs. In some embodiments, the ECs are adult ECs. In some embodiments, the ECs are not embryonic ECs. In some embodiments, the ECs are human ECs. In some embodiments, the ECs are primary ECs. In some embodiments, the ECs are human umbilical vein ECs (HUVECs).

In some embodiments, the initial EC/HSPC ratio is at least about 200: 1. In some embodiments, the initial EC/HSPC ratio is at least about 300: 1. In some embodiments, the initial EC/HSPC ratio is at least about 400: 1. In some embodiments, the initial EC/HSPC ratio is at least about 500:1. In some embodiments, the initial EC/HSPC ratio is at least about 600: 1. In some embodiments, the initial EC/HSPC ratio is at least about 700: 1. In some embodiments, the initial EC/HSPC ratio is at least about 800:1. In some embodiments, the initial EC/HSPC ratio is at least about 900:1. In some embodiments, the initial EC/HSPC ratio is at least about 1000:1. In some embodiments, the initial EC/HSPC ratio is at least about 1100: 1. In some embodiments, the initial EC/HSPC ratio is at least about 1200: 1. In some embodiments, the initial EC/HSPC ratio is at least about 1300: 1. In some embodiments, the initial EC/HSPC ratio is at least about 1400: 1. In some embodiments, the initial EC/HSPC ratio is about 1500:1. In some embodiments, the initial EC/HSPC ratio is about 2000:1. In some embodiments, the initial EC/HSPC ratio is about 3000:1.

In some embodiments, the first time period is about 1 day to about 24 days. In some embodiments, the first time period is about 1 day to about 12 days. In some embodiments, the first time period is about 1 day. In some embodiments, the first time period is about 2 days. In some embodiments, the first time period is about 3 days. In some embodiments, the first time period is about 4 days. In some embodiments, the first time period is about 5 days. In some embodiments, the first time period is about 6 days. In some embodiments, the first time period is about 7 days. In some embodiments, the first time period is about 8 days. In some embodiments, the first time period is about 9 days. In some embodiments, the first time period is about 10 days. In some embodiments, the first time period is about 11 days. In some embodiments, the first time period is about 12 days. In some embodiments, the first time period is about 13 day. In some embodiments, the first time period is about 14 days. In some embodiments, the first time period is about 15 days. In some embodiments, the first time period is about 16 days. In some embodiments, the first time period is about 17 days. In some embodiments, the first time period is about 18 days. In some embodiments, the first time period is about 19 days. In some embodiments, the first time period is about 20 days. In some embodiments, the first time period is about 21 days. In some embodiments, the first time period is about 22 days. In some embodiments, the first time period is about 23 days. In some embodiments, the first time period is about 24 days. In some embodiments, the first time period is about 25 days. In some embodiments, the first time period is about 26 days. In some embodiments, the first time period is about 27 days. In some embodiments, the first time period is about 28 days.

In some embodiments, the expanded HSPC/EC ratio is about 10:1 to about 1:10. In some embodiments, the expanded HSPC/EC ratio about 9:1 to about 1:9. In some embodiments, the expanded HSPC/EC ratio is about 8:1 to about 1:8. In some embodiments, the expanded HSPC/EC ratio is about 7:1 to about 1:7. In some embodiments, the expanded HSPC/EC ratio is about 6:1 to about 1:6. In some embodiments, the expanded HSPC/EC ratio is about 5:1 to about 1:5. In some embodiments, the expanded HSPC/EC ratio is about 4:1 to about 1:4. In some embodiments, the expanded HSPC/EC ratio is about 3:1 to about 1:3. In some embodiments, the expanded HSPC/EC ratio is about 2:1 to about 1:2. In some embodiments, the expanded HSPC/EC ratio is about 10:1 to about 1:1. In some embodiments, the expanded HSPC/EC ratio is about 9:1 to about 2:1. In some embodiments, the expanded HSPC/EC ratio is about 8:1 to about 3:1. In some embodiments, the expanded HSPC/EC ratio is about 7:1 to about 4:1. In some embodiments, the expanded HSPC/EC ratio is about 6:1 to about 5:1. In some embodiments, the expanded HSPC/EC ratio is about 10:1. In some embodiments, the expanded HSPC/EC ratio is about 9:1. In some embodiments, the expanded HSPC/EC ratio is about 8:1. In some embodiments, the expanded HSPC/EC ratio is about 7:1. In some embodiments, the expanded HSPC/EC ratio is about 6:1. In some embodiments, the expanded HSPC/EC ratio is about 5:1. In some embodiments, the expanded HSPC/EC ratio is about 4:1. In some embodiments, the expanded HSPC/EC ratio is about 3:1. In some embodiments, the expanded HSPC/EC ratio is about 2:1. In some embodiments, the expanded HSPC/EC ratio is about 1:1.

In some embodiments, the HSPCs expand by about 20-fold to about 30,000-fold during the expansion step. In some embodiments, the HSPCs expand by about 1,000-fold to about 10,000-fold during the expansion step. In some embodiments, the HSPCs expand by about 1,000-fold during the expansion step. In some embodiments, the HSPCs expand by about 2,000-fold during the expansion step. In some embodiments, the HSPCs expand by about 3,000-fold during the expansion step. In some embodiments, the HSPCs expand by about 3,500-fold during the expansion step. In some embodiments, the HSPCs expand by about 4,000-fold during the expansion step. In some embodiments, the HSPCs expand by about 5,000-fold during the expansion step. In some embodiments, the HSPCs expand by about 6,000-fold during the expansion step. In some embodiments, the HSPCs expand by about 7,000-fold during the expansion step. In some embodiments, the HSPCs expand by about 8,000-fold during the expansion step. In some embodiments, the HSPCs expand by about 9,000-fold during the expansion step. In some embodiments, the HSPCs expand by about 10,000-fold during the expansion step.

In some embodiments, the continuous interaction between the HSPCs and the ECs comprises continuous contact between the HSPCs and the ECs during the expansion step, and continuous proximity of the HSPCs and the ECs during the expansion and transfusion steps combined. In some embodiments, the methods described herein further comprise harvesting the expanded cell population and preparing a pharmaceutical composition comprising the expanded cell population for use in the transfusion step. In some embodiments, the continuous interaction between the HSPCs and the ECs comprises continuous proximity of the HSPCs and the ECs during the expansion, harvesting, pharmaceutical composition preparation, and transfusion steps combined. In some embodiments the methods described herein do not comprise a purification step whereby the endothelial cells are separated from the expanded HSPCs prior to transfusion. In some embodiments there is no human interaction or disturbance of the expansion during the first time period. In some embodiments, supplemental cytokines are added during the first time period. In some embodiments, 50 ng/mL of thrombopoietin (TPO), Flt3 (FMS-related tyrosine kinase 3) ligand, and stem cell factor (SCF) are added during the first time period. In some embodiments the expansion step comprises not more than six open events.

Prior to expansion, the HSPCs and ECs may subjected to selection and purification, which can include both positive and negative selection methods, to obtain a substantially pure population of cells. In one aspect, fluorescence activated cell sorting (FACS), also referred to as flow cytometry, is used to sort and analyze different cell populations. Cells having the cellular markers specific for HSPC or EC populations are tagged with an antibody, or typically a mixture of antibodies, that bind the cellular markers. Each antibody directed to a different marker is conjugated to a detectable molecule, particularly a fluorescent dye that can be distinguished from other fluorescent dyes coupled to other antibodies. A stream of tagged or "stained" cells is passed through a light source that excites the fluorochrome and the emission spectrum from the cells detected to determine the presence of a particular labeled antibody. By concurrent detection of different fluorochromes, also referred to in the art as multicolor fluorescence cell sorting, cells displaying different sets of cell markers may be identified and isolated from other cells in the population. Other FACS parameters, including, by way of example and not limitation, side scatter (SSC), forward scatter (FSC), and vital dye staining (*e.g*., with propidium iodide) allow selection of cells based on size and viability. FACS sorting and analysis of HSPCs is described in, among others, U.S. Patent Nos. 5,137,809, 5,750,397, 5,840,580; 6,465,249; Manz, M.G. et al., Proc. Natl. Acad. Sci. USA 99:11872-11877 (2002); and Akashi, K. et al., Nature 404(6774):193-197 (2000)). General guidance on fluorescence activated cell sorting is described in, for example, Shapiro, H.M., Practical Flow Cytometry, 4th Ed., Wiley-Liss (2003) and Ormerod, M.G., Flow Cytometry: A Practical Approach, 3rd Ed., Oxford University Press (2000).

It is to be understood that the purification of cells also includes combinations of the methods described herein. A typical combination may comprise an initial procedure that is effective in removing the bulk of unwanted cells and cellular material, for example leukapharesis. A second step may include isolation of cells expressing a marker common to one or more of the progenitor cell populations by immunoadsorption on antibodies bound to a substrate. For example, magnetic beads containing anti-CD34 antibodies are able to bind and capture HSC, CMP, and GMP cells that commonly express the CD34 antigen. An additional step providing higher resolution of different cell types, such as FACS sorting with antibodies to a set of specific cellular markers, can be used to obtain substantially pure populations of the desired cells. Another combination may involve an initial separation using magnetic beads bound with anti-CD34 antibodies followed by an additional round of purification with FACS.

Venues for accomplishing the expansion are well known to those of skill in the art and include, for example, open cell culture environments, such as culture flasks, and closed cell culture environments such as closed-system wave bag bioreactors with or without modification for cell adhesion, and closed-system mini-bioreactors, for example mini-bioreactors comprising hollow fibers or other systems or coatings allowing adhesion of cells if desired. In some embodiments, the venue is the Quantum^{®} Cell Expansion System (Terumo BCT, Lakewood, CO).

The amount of the cells needed for achieving a therapeutic effect will be determined empirically in accordance with conventional procedures for the particular purpose. Generally, for administering cells for therapeutic purposes, the cells are given at a pharmacologically effective dose. By "pharmacologically effective amount" or "pharmacologically effective dose" is meant an amount sufficient to produce the desired physiological effect or amount capable of achieving the desired result, particularly for engraftment or survival of a subject. Therapeutic benefit also includes halting or slowing the progression of the underlying disease or disorder, regardless of whether improvement is realized. Pharmacologically effective dose, as defined above, will also apply to therapeutic compounds used in combination with the cells, as further described below.

The hematopoietic stem cell transplant graft can vary widely as a function of the age, weight and state of health of the patient, the nature and the severity of the indication. Suitable dosage ranges for the HSPCs vary according to these considerations. In some embodiments, a graft will contain about 1 × 10⁶ to about 1.0 × 10⁸ HSPCs/kg subject. In some embodiments, the expanded cell population comprises about 1.0 × 10⁶ to about 1.0 × 10⁸ HSPCs/kg subject. In some embodiments, the expanded cell population comprises about 1.0 × 10⁶ HSPCs/kg subject. In some embodiments, the expanded cell population comprises about 2.0 × 10⁶ HSPCs/kg subject. In some embodiments, the expanded cell population comprises about 3.0 × 10⁶ HSPCs/kg subject. In some embodiments, the expanded cell population comprises about 4.0 × 10⁶ HSPCs/kg subject. In some embodiments, the expanded cell population comprises about 5.0 × 10⁶ HSPCs/kg subject. In some embodiments, the expanded cell population comprises about 6.0 × 10⁶ HSPCs/kg subject. In some embodiments, the expanded cell population comprises about 7.0 × 10⁶ HSPCs/kg subject. In some embodiments, the expanded cell population comprises about 8.0 × 10⁶ HSPCs/kg subject. In some embodiments, the expanded cell population comprises about 9.0 × 10⁶ HSPCs/kg subject. In some embodiments, the expanded cell population comprises about 1.0 × 10⁷ HSPCs/kg subject. In some embodiments, the expanded cell population comprises about 2.0 × 10⁷ HSPCs/kg subject. In some embodiments, the expanded cell population comprises about 3.0 × 10⁷ HSPCs/kg subject. In some embodiments, the expanded cell population comprises about 4.0 × 10⁷ HSPCs/kg subject. In some embodiments, the expanded cell population comprises about 5.0 × 10⁷ HSPCs/kg subject. In some embodiments, the expanded cell population comprises about 6.0 × 10⁷ HSPCs/kg subject. In some embodiments, the expanded cell population comprises about 7.0 × 10⁷ HSPCs/kg subject. In some embodiments, the expanded cell population comprises about 8.0 × 10⁷ HSPCs/kg subject. In some embodiments, the expanded cell population comprises about 9.0 × 10⁷ HSPCs/kg subject. In some embodiments, the expanded cell population comprises about 1.0 × 10⁸ HSPCs/kg subject.

A cord blood unit is the blood collected from a single placenta and umbilical cord. The number of nucleated cells in a cord blood unit varies. In addition, the number of nucleated cells in a cord blood unit may be less after freezing and thawing. Thus, in administering HSPCs, it is instructive to note whether nucleated cell count was measured before or after thawing the unit.

Transplantation of cells into patient is accomplished by methods generally used in the art. The preferred method of administration is intravenous infusion. As described above, the number of cells transfused will take into consideration factors such as sex, age, weight, the types of disease or disorder, stage of the disorder, the percentage of the desired cells in the cell population (*e.g*., purity of cell population), and the cell number needed to produce a therapeutic benefit.

Cells can be administered in one infusion, or through successive infusions over a defined time period sufficient to generate a therapeutic effect. Different populations of cells may be infused when treatment involves successive infusions. A pharmaceutically acceptable carrier, as further described below, may be used for infusion of the cells into the patient. These will typically comprise, for example, buffered saline (*e.g.*, phosphate buffered saline) or un-supplemented basal cell culture medium, or medium as known in the art.

The methods of hematopoietic stem cell transplantation described herein can be used for treatment of various disorders. In some embodiments, the disorder relates to deficiencies in hematopoiesis caused by disease or myeloablative treatments. In some embodiments, the disorder is a hematopoietic disease. In some embodiments, the disorder is a disorder that requires bone marrow hematopoietic stem cell transplantation. In some embodiments, the disorder is an infectious or genetic immunodeficiency. In some embodiments, the disorder is an infectious immunodeficiency. In some embodiments, the disorder is HIV. In some embodiments, the disorder is a genetic immunodeficiency. In some embodiments, the disorder is a hematologic disease, including infectious diseases/genetic diseases affecting T cells (*e.g.,* HIV, SCID, respectively) and genetic diseases affecting erythrocytes (hemoglobinopathies). In some embodiments, the disorder is an anemia. In some embodiments, the disorder is Fanconi anemia. In some embodiments, the disorder is a hematopoietic malignancy. As used herein, "treatment" refers to therapeutic or prophylactic treatment, or a suppressive measure for the disease, disorder or undesirable condition. Treatment encompasses administration of the subject cells in an appropriate form prior to the onset of disease symptoms and/or after clinical manifestations, or other manifestations of the disease or condition to reduce disease severity, halt disease progression, or eliminate the disease. Prevention of the disease includes prolonging or delaying the onset of symptoms of the disorder or disease, preferably in a subject with increased susceptibility to the disorder. In some embodiments, the subject is human.

The disclosure further provides enhanced methods of HSCT in the field of solid organ, cell, or tissue transplantation. By way of example, but not as a limitation, the disclosure further provides endothelial cell-expanded HSPCs for use in the transplant of heart, lung liver, kidney, islet cells, skin, endocrine organs, or pancreas.

In some embodiments, the platelet count in the circulating blood of the subject is at least about 20,000/µL by about 15 days to about 35 days after transfusing the expanded cell population into the subject. In some embodiments, the platelet count in the circulating blood of the subject is at least about 20,000/µL by about 20 days to about 30 days after transfusing the expanded cell population into the subject. In some embodiments, the platelet count in the circulating blood of the subject is at least about 20,000/µL by about 20 days after transfusing the expanded cell population into the subject. In some embodiments, the platelet count in the circulating blood of the subject is at least about 20,000/µL by about 21 days after transfusing the expanded cell population into the subject. In some embodiments, the platelet count in the circulating blood of the subject is at least about 20,000/µL by about 22 days after transfusing the expanded cell population into the subject. In some embodiments, the platelet count in the circulating blood of the subject is at least about 20,000/µL by about 23 days after transfusing the expanded cell population into the subject. In some embodiments, the platelet count in the circulating blood of the subject is at least about 20,000/µL by about 24 days after transfusing the expanded cell population into the subject. In some embodiments, the platelet count in the circulating blood of the subject is at least about 20,000/µL by about 25 days after transfusing the expanded cell population into the subject. In some embodiments, the platelet count in the circulating blood of the subject is at least about 20,000/µL by about 26 days after transfusing the expanded cell population into the subject. In some embodiments, the platelet count in the circulating blood of the subject is at least about 20,000/µL by about 27 days after transfusing the expanded cell population into the subject. In some embodiments, the platelet count in the circulating blood of the subject is at least about 20,000/µL by about 28 days after transfusing the expanded cell population into the subject. In some embodiments, the platelet count in the circulating blood of the subject is at least about 20,000/µL by about 29 days after transfusing the expanded cell population into the subject. In some embodiments, the platelet count in the circulating blood of the subject is at least about 20,000/µL by about 30 days after transfusing the expanded cell population into the subject.

In some embodiments, the absolute neutrophil count in the circulating blood of the subject is at least about 100/µL by about 5 days to about 20 days after transfusing the expanded cell population into the subject. In some embodiments, the absolute neutrophil count in the circulating blood of the subject is at least about 100/µL by about 5 days after transfusing the expanded cell population into the subject. In some embodiments, the absolute neutrophil count in the circulating blood of the subject is at least about 100/µL by about 6 days after transfusing the expanded cell population into the subject. In some embodiments, the absolute neutrophil count in the circulating blood of the subject is at least about 100/µL by about 7 days after transfusing the expanded cell population into the subject. In some embodiments, the absolute neutrophil count in the circulating blood of the subject is at least about 100/µL by about 8 days after transfusing the expanded cell population into the subject. In some embodiments, the absolute neutrophil count in the circulating blood of the subject is at least about 100/µL by about 9 days after transfusing the expanded cell population into the subject. In some embodiments, the absolute neutrophil count in the circulating blood of the subject is at least about 100/µL by about 10 days after transfusing the expanded cell population into the subject. In some embodiments, the absolute neutrophil count in the circulating blood of the subject is at least about 100/µL by about 11 days after transfusing the expanded cell population into the subject. In some embodiments, the absolute neutrophil count in the circulating blood of the subject is at least about 100/µL by about 12 days after transfusing the expanded cell population into the subject. In some embodiments, the absolute neutrophil count in the circulating blood of the subject is at least about 100/µL by about 13 days after transfusing the expanded cell population into the subject. In some embodiments, the absolute neutrophil count in the circulating blood of the subject is at least about 100/µL by about 14 days after transfusing the expanded cell population into the subject. In some embodiments, the absolute neutrophil count in the circulating blood of the subject is at least about 100/µL by about 15 days after transfusing the expanded cell population into the subject. In some embodiments, the absolute neutrophil count in the circulating blood of the subject is at least about 100/µL by about 16 days after transfusing the expanded cell population into the subject. In some embodiments, the absolute neutrophil count in the circulating blood of the subject is at least about 100/µL by about 17 days after transfusing the expanded cell population into the subject. In some embodiments, the absolute neutrophil count in the circulating blood of the subject is at least about 100/µL by about 18 days after transfusing the expanded cell population into the subject. In some embodiments, the absolute neutrophil count in the circulating blood of the subject is at least about 100/µL by about 19 days after transfusing the expanded cell population into the subject. In some embodiments, the absolute neutrophil count in the circulating blood of the subject is at least about 100/µL by about 20 days after transfusing the expanded cell population into the subject.

In some embodiments, the absolute neutrophil count in the circulating blood of the subject is at least about 500/µL by about 10 days to about 25 days after transfusing the expanded cell population into the subject. In some embodiments, the absolute neutrophil count in the circulating blood of the subject is at least about 500/µL by about 10 days after transfusing the expanded cell population into the subject. In some embodiments, the absolute neutrophil count in the circulating blood of the subject is at least about 500/µL by about 11 days after transfusing the expanded cell population into the subject. In some embodiments, the absolute neutrophil count in the circulating blood of the subject is at least about 500/µL by about 12 days after transfusing the expanded cell population into the subject. In some embodiments, the absolute neutrophil count in the circulating blood of the subject is at least about 500/µL by about 13 days after transfusing the expanded cell population into the subject. In some embodiments, the absolute neutrophil count in the circulating blood of the subject is at least about 500/µL by about 14 days after transfusing the expanded cell population into the subject. In some embodiments, the absolute neutrophil count in the circulating blood of the subject is at least about 500/µL by about 15 days after transfusing the expanded cell population into the subject. In some embodiments, the absolute neutrophil count in the circulating blood of the subject is at least about 500/µL by about 16 days after transfusing the expanded cell population into the subject. In some embodiments, the absolute neutrophil count in the circulating blood of the subject is at least about 500/µL by about 17 days after transfusing the expanded cell population into the subject. In some embodiments, the absolute neutrophil count in the circulating blood of the subject is at least about 500/µL by about 18 days after transfusing the expanded cell population into the subject. In some embodiments, the absolute neutrophil count in the circulating blood of the subject is at least about 500/µL by about 19 days after transfusing the expanded cell population into the subject. In some embodiments, the absolute neutrophil count in the circulating blood of the subject is at least about 500/µL by about 20 days after transfusing the expanded cell population into the subject. In some embodiments, the absolute neutrophil count in the circulating blood of the subject is at least about 500/µL by about 21 days after transfusing the expanded cell population into the subject. In some embodiments, the absolute neutrophil count in the circulating blood of the subject is at least about 500/µL by about 22 days after transfusing the expanded cell population into the subject. In some embodiments, the absolute neutrophil count in the circulating blood of the subject is at least about 500/µL by about 23 days after transfusing the expanded cell population into the subject. In some embodiments, the absolute neutrophil count in the circulating blood of the subject is at least about 500/µL by about 24 days after transfusing the expanded cell population into the subject. In some embodiments, the absolute neutrophil count in the circulating blood of the subject is at least about 500/µL by about 25 days after transfusing the expanded cell population into the subject.

In some embodiments, the absolute neutrophil count in the circulating blood of the subject is at least about 500/µL at about 100 days after transfusing the expanded cell population into the subject. In some embodiments, the absolute neutrophil count in the circulating blood of the subject is at least about 500/µL at about 200 days after transfusing the expanded cell population into the subject. In some embodiments, the absolute neutrophil count in the circulating blood of the subject is at least about 500/µL at about 300 days after transfusing the expanded cell population into the subject. In some embodiments, the absolute neutrophil count in the circulating blood of the subject is at least about 500/µL at about 400 days after transfusing the expanded cell population into the subject. In some embodiments, the absolute neutrophil count in the circulating blood of the subject is at least about 500/µL at about 500 days after transfusing the expanded cell population into the subject. In some embodiments, the absolute neutrophil count in the circulating blood of the subject is at least about 500/µL at about 600 days after transfusing the expanded cell population into the subject. In some embodiments, the absolute neutrophil count in the circulating blood of the subject is at least about 500/µL at about 700 days after transfusing the expanded cell population into the subject. In some embodiments, the absolute neutrophil count in the circulating blood of the subject is at least about 500/µL at about 800 days after transfusing the expanded cell population into the subject. In some embodiments, the absolute neutrophil count in the circulating blood of the subject is at least about 500/µL at about 900 days after transfusing the expanded cell population into the subject. In some embodiments, the absolute neutrophil count in the circulating blood of the subject is at least about 500/µL at about 1,000 days after transfusing the expanded cell population into the subject.

### Adjunctive Treatments

A variety of adjunctive treatments may be used with the methods described herein. In one aspect, the adjunctive treatments include, among others, anti-fungal agents, anti-bacterial agents, and anti-viral agents.

In one aspect, the adjunctively administered agent is an anti-fungal agent. Fungal infections are a significant problem in patients who have undergone myeloablative therapy and HSCT. Recipients with delayed engraftment and patients who develop GVHD typically are at high risk for fungal infections. Types of fungal infections are varied, and include, among others, candidiasis (*e.g.,* with *Candida krusei, Candida glabrata, Candida albicans, Candida tropicalis*), aspergillosis (*e.g.,* with *Aspergillus fumigatus*, *Aspergillus flavus*), mucormycosis (*e.g.,* with *Rhizobium arrhizus*, *Absidia corymbifera*, *Rhizomucor pusillus*), cryptococcosis, *Histoplasma capsulatum,* and *Coccidioides immitis.*

Anti-fungal agents for adjunctive administration will generally be a systemic antifungal agent. One useful antifungal agent of this type is amphotericin B from the family of polyene macrolide antibiotics. Amphotericin B is available in various formulations, including as a complex with deoxycholate; in a colloidal suspension with cholestearyl sulfate; and encapsulated in liposomes made of soy lecithin, cholesterol, and distearoylphosphatidylglycerol, other formulations are known in the art.

Another antifungal agent is flucytosine, a fluorinated pyrimidine. Deamination of flucytosine by the fungus generates 5-flurouracil, an anti-metabolite and DNA synthesis inhibitor. Flucytosine is typically used for infections of cryptococcus and candiadosis. Although used alone, flucytosine is generally used in combination with amphotericin B.

Imidazoles and triazoles represent a broad class of azole based antifungal agents. It is believed that imidazoles and triazoles inhibit sterol 14-α-demethylase, resulting in impaired biosynthesis of ergosterol and disruption of cell membrane based activities, such as electron transport. Azole based anti-fungals are effective against certain types of candiadosis, such as *Candida albicans, Candida glabrata,* and *Candida neoformans*. Exemplary azole antifungals suitable for systemic administration include, among others, ketoconzaole, itracanazole, fluconazole, econazole, voriconazole, and tercanozole.

In addition to fungal infections, a patient with neutropenia is susceptible to infection with a variety of bacterial pathogens. Patients undergoing myeloablative regimens and HSCT have high rates of bacterial infection with both Gram positive (*e.g., Streptococcus* and *Staphylococcus aureus*) and Gram negative bacteria (*e.g., E. coli* and *Pseudomonas aeruginosa*)*.* Septecemia is a common occurrence. In addition, delayed engraftment and impaired restoration of immune responses against encapsulated bacteria, such as *Streptococcus pneumoniae* or *Haemophilus influenza,* increases the morbidity rate for transplant recipients with GVHD.

Adjunctive antibacterial therapy can use any known antibiotics suitable for the particular bacterial pathogen. These include both wide spectrum antibiotics and more targeted anti-bacterial compounds. Various classes of anti-bacterial agents suitable with the expanded myeloid cells include, by way of example and not limitation, quinolones and fluoroquinolones, β-lactam antibiotics, aminoglycosides, tetracyclins, macrolides, and various cogeners thereof. Exemplary quinolone compounds include ciprofloxacin, ofloxacin, sparfloxacin, lomefloxacin, and moxifloxacin. Exemplary β-lactam antibiotics include penicillins (*e.g*., penicillin G, penicillin V), ampicillin, carbenicillin, methicillin, carbapenem, and cephalosporins (*e.g*., cephalothin, cefamandole, cefaclor, cefonicid, cefotetan, cefatoxime, ceftazidime, ceftizoxime, cefepime). Exemplary aminoglycosides include neomycin, streptomycin, kanamycin, gentamicin, tobramycin, amikacin, and netilmicin. Exemplary macrolides include erythromycin, clarithromycin, azithromycin, and solithromycin. Other antibiotics will be apparent to the skilled artisan.

Viral infections are also problematic in myeloablated patients and HSCTs. Generally the increased risk of viral infection results from impaired cell mediated immunity brought on by the myeloablative therapy. Many of these infections arise from reactivation of latent virus existing in a seropositive patient or in the cells of a seropositive donor. Viruses commonly encountered include, among others, cytomegalovirus, herpes simplex virus, varicella zoster virus, herpesvirus-6, Epstein Barr virus, adenoviruses, and the like. As an adjunct to the cell infusions, anti-viral compounds selected are those appropriate to the viruses encountered by the patient. Useful antiviral compounds include, by way of example and not limitation, acyclovir, cidofovir, ganciclovir, idoxuridine, penciclovir, valganciclovir, valacyclovir, vidarabine, amantadine, rimantadine, zanamivir, fomivirsen, imiquimod, and ribavirin. Therapeutics directed against retroviruses include, among others, nucleoside reverse transcriptase inhibitors (*e.g*., zidovudine, didanosine, stavudine, zalcitabine, lamividudine), non-nucleoside reverse transcriptase inhibitors (*e.g.,* nevirapine, efavirenz, delvirudine), and protease inhibitors (*e.g.,* saquinivir, indinavir, ritonavir, nelfinavir, amprenavir, and lopinavir).

The antifungal, antibacterial, and antiviral agents may be used as prophylaxis to reduce the occurrence of the infection, or upon appearance of the disease. Prophylaxis is particularly indicated for fungal infections common in immunosuppressed patients, and for viral infections in seropositive patients or seropositive transplant donors. Accordingly, embodiments for therapeutic purposes include combinations of HSPCs, MP cells and the antifungal, antibacterial, or antiviral compounds.

In a further embodiment, the adjunctively administered agent is a cytokine or growth factor that enhances differentiation and mobilization of terminally differentiated myeloid cells, particularly granulocytes, macrophages, megakaryocytes and erythroid cells. For enhancing granulocyte development, the cytokines C-CSF and GM-CSF may be used. G-CSF is effective in accelerating engraftment and production of neutrophils in HSCT. In another embodiment, the cytokine or growth factor is thrombopoietin. Administration of TPO enhances engraftment of transplanted progenitor cells and promotes development of megakaryocytes and platelets (Fox, N et al., J. Clin. Invest. 110:389-394 (2002); Akahori, H. et al., Stem Cells 14(6):678-689 (1996)).

A variety of vehicles and excipients and routes of administration may be used for adjunctive therapy, as will be apparent to the skilled artisan. Representative formulation technology is taught in, inter alia, Remington: The Science and Practice of Pharmacy, 19th Ed., Mack Publishing Co., Easton, PA (1995) and Handbook of Pharmaceutical Excipients, 3rd Ed, Kibbe, A.H. ed., Washington DC, American Pharmaceutical Association (2000).

The pharmaceutical compositions will generally comprise a pharmaceutically acceptable carrier and a pharmacologically effective amount of the compounds, or mixture of thereof, or suitable salts thereof. The pharmaceutical composition may be formulated as powders, granules, solutions, suspensions, aerosols, solids, pills, tablets, capsules, gels, topical crèmes, suppositories, transdermal patches, and other formulations known in the art.

As used herein, "pharmaceutically acceptable carrier" comprises any of standard pharmaceutically accepted carriers known to those of ordinary skill in the art in formulating pharmaceutical compositions. Thus, the compounds, by themselves, such as being present as pharmaceutically acceptable salts, or as conjugates, may be prepared as formulations in pharmaceutically acceptable diluents; for example, saline, phosphate buffer saline (PBS), aqueous ethanol, or solutions of glucose, mannitol, dextran, propylene glycol, oils (*e.g.,* vegetable oils, animal oils, synthetic oils, *etc*.), microcrystalline cellulose, carboxymethyl cellulose, hydroxylpropyl methyl cellulose, magnesium stearate, calcium phosphate, gelatin, polysorbate 80 or the like, or as solid formulations in appropriate excipients.

The pharmaceutical compositions will often further comprise one or more buffers (*e.g.,* neutral buffered saline or phosphate buffered saline), carbohydrates (*e.g*., glucose, mannose, sucrose or dextrans), mannitol, proteins, polypeptides or amino acids such as glycine, antioxidants (*e.g*., ascorbic acid, sodium metabisulfite, butylated hydroxytoluene, butylated hydroxyanisole, *etc*.), bacteriostats, chelating agents such as EDTA or glutathione, solutes that render the formulation isotonic, hypotonic or weakly hypertonic with the blood of a recipient, suspending agents, thickening agents, preservatives, flavoring agents, sweetening agents, and coloring compounds as appropriate.

While any suitable carrier known to those of ordinary skill in the art may be employed in the compositions, the type of carrier will typically vary depending on the mode of administration. Therapeutic compositions may be formulated for any appropriate manner of administration, including for example, oral, nasal, mucosal, rectal, vaginal, topical, intravenous, intraperitoneal, intradermal, subcutaneous, and intramuscular administration.

The pharmaceutical compositions described herein may be presented in unit-dose or multi-dose containers, such as sealed ampoules or vials. Such containers are typically sealed in such a way to preserve the sterility and stability of the formulation until use. In general, formulations may be stored as suspensions, solutions or emulsions in oily or aqueous vehicles, as indicated above. Alternatively, a pharmaceutical composition may be stored in a freeze-dried condition requiring only the addition of a sterile liquid carrier immediately prior to use.

The amount administered to the host will vary depending upon what is being administered, the purpose of the administration, such as prophylaxis or therapy, the state of the host, the manner of administration, the number of administrations, interval between administrations, and the like. These can be determined empirically by those skilled in the art and may be adjusted for the extent of therapeutic response. Factors to consider in determining an appropriate dose include, but is not limited to, size and weight of the subject, the age and sex of the subject, the severity of the symptom, the stage of the disease, method of delivery of the agent, half-life of the agents, and efficacy of the agents. Stage of the disease to consider includes whether the disease is acute or chronic, relapsing or remitting phase, and the progressiveness of the disease.

Determining the dosages and times of administration for a therapeutically effective amount are well within the skill of the ordinary person in the art. For example, an initial effective dose can be estimated from cell culture or other *in vitro* assays. A dose can then be formulated in animal models to generate a circulating concentration or tissue concentration, including that of the IC₅₀ as determined by the cell culture assays.

### Kits disclosed but not part of invention

The methods described herein can be facilitated by a kit for enhancing HSPC engraftment. The kits may contain cells including but not limited to HSPCs, ECs, including expanded and or isolated cells, and/or adjunctive therapeutic compounds, means for isolating or expanding HSPCs and ECs, means for administering cells to a patient, or any combination thereof. The kits may optionally comprise any or all of a pharmaceutically acceptable carrier, a physiologically acceptable carrier, instructions for use, a container, a vessel for administration, antibodies, or any combination thereof. A label typically accompanies the kit, and includes any writing or recorded material, which may be electronic or computer readable form (*e.g.,* disk, optical disc, memory chip, or tape) providing instructions or other information for use of the kit contents.

The practice of the present invention will employ, unless otherwise indicated conventional techniques of cell biology, molecular biology, cell culture, immunology and the like which are in the skill of one in the art. These techniques are fully disclosed in the current literature and reference is made specifically to Sambrook, Fritsch and Maniatis eds., "Molecular Cloning A Laboratory Manual, 2nd Ed., Cold Springs Harbor Laboratory Press, 1989); the series Methods of Enzymology (Academic Press, Inc.); and Antibodies: A Laboratory Manual, Harlow et al., eds., (1987).

### EXAMPLES

Aspects of the present teachings may be further understood in light of the following examples, which should not be construed as limiting the scope of the present teachings in any way.

### Example 1: Expansion of HSPCs with Non-irradiated ECs

Non-irradiated E4ORF1+ engineered ECs have the proliferative capacity to be passaged at 1:4 splits approximately 15-20 times in traditional flask based culture. In this method, non-irradiated E4ORF1+ engineered ECs can be expanded to sufficient quantities in flat-surfaced culture vessels to confluence. The amount of cells plated will depend on the amount of starting material and the length of the expansion. For example, a 10 day expansion may require the equivalent of 3 plates worth of 6-well dishes (each approximately 35 mm in diameter). E4ORF1+ ECs are cultured in complete growth medium until confluence in these wells. At confluence, the media is transitioned to conditioning media. After 24 hours in conditioning media, CD34+ purified cells, for example from umbilical cord blood (UCB), can be seeded on the E4ORF1+ EC-pre-seeded wells with cytokines, consisting of 50ng/ml each of thrombopoietin (TPO), Fms-related tyrosine kinase 3 ligand (Flt-3L), and stem cell factor (SCF). After two days, the media is harvested, cells are pelleted and re-suspended in fresh cytokine containing media and dispersed into two additional wells that have been preconditioned as the first. The first well, referred to as the "mother well" or "mother flask," is also re-fed with additional cytokine containing media. This process is repeated every other day until the 10^{th} day. At this point, all wells contain expanded HSPCs and E4ORF1+ ECs. In some cases a bioreactor, such as a hollow fiber bioreactor, may be utilized. The hollow fibers can be primed, coated, conditioned (for example, as per the manufacturer's instructions), and seeded with approximately 5x10⁷ E4ORF1+ ECs in complete growth medium. The bioreactor "feeds" the cells and may allow expansion for 6 days. At this point, with the bioreactor near confluence, the media is replaced with conditioning media without cytokines to displace the serum (e.g. fetal bovine serum (FBS)), which could otherwise drive premature differentiation of the HSPCs. After 24 hours, the E4ORF1+ ECs are ready to accept HSPCs. UCB samples can be stored frozen as red blood cell (RBC) depleted product in dimethyl sulfoxide (DMSO). This cellular material is washed prior to purification of a primitive CD34+ population and expansion. In order to achieve this, the sample is thawed in a 37°C bead bath. Upon visual inspection for remaining ice, the unit is sterile-welded to the fluidics of a Miltenyi Prodigy device or a similar system. The cells are incubated at this time with DNAse, as the nucleic acid content of any non-viable cells post thaw may result in clumping and viscosity. The Miltenyi Prodigy device, or comparable device, is capable of washing the cells to remove the cryopreservative and further depleting the sample of RBCs. While still in the Miltenyi Prodigy device, the cells are incubated with CD34 microbeads to purify the HSPCs. Finally, washing steps are performed. The microbead-bound CD34+ cells are then injected into a Miltenyi CliniMacs device or similar device for magnetic purification of the labeled CD34⁺ cells. Recovery of 50-70% of the available CD34⁺ cells is expected.

The purified CD34+ fraction is then injected into a total of 200mls of HSPC expansion media supplemented with cytokines, SCF, TPO, and Flt3L at 50ng/ml each. This milieu is then introduced into the bioreactor in the same interior chamber as the E4ORF1+ engineered ECs (Day 0 of the expansion). Throughout the expansion, HSPC expansion media (without cytokines) is continuously supplied via the exterior chamber at a rate of approximately 0.4ml/min for the exchange of metabolites and circulates at a rate of approximately 30ml/min in a recycling loop, which includes a gas exchange module supplied with 90% N₂, 5% O₂, and 5% CO₂. Additionally, on Day 2 and Day 4 of the expansion, a bolus of 200mls of HSPC expansion media supplemented with cytokines, SCF, TPO, and Flt3L at 50ng/ml each is added into the interior chamber. The expansion is stopped after on Day 6 of the expansion, at which point the entirety of the cellular material is harvested.

### Example 2: Expansion of HSPCs with Irradiated ECs

E4ORF1+ engineered ECs are fully competent to expand HSPCs after receiving a mitotic inactivation stimulus, such as radiation or mitomycin C. To utilize the cells after mitotic inactivation, the mitotic inactivation stimulus is administered when the cells are in their final culture vessel at confluence and allowed 24 hours to recover in complete media prior to switching to the conditioning media. For use of mitotically inactivated cells in bioreactors, E4ORF1+ engineered ECs, or ETV2+ E4ORF1+ engineered ECs, or E4ORF1+ E4ORF6+ engineered ECs, are grown until there are approximately 1 billion cells. The cells may be cryopreserved after treatment with the mitotic inactivation stimulus. The entirety of the mitotically-inactivated EC sample is then injected into the bioreactor and allowed to attach for 24 hours. Conditioning of the media follows for an additional 24 hours. CD34+ cells, from UCB for example, are then introduced into the same compartment of the bioreactor as the E4ORF1+ engineered ECs supplemented with cytokines (50ng/ml TPO, Flt-3L, SCF). From this point on, the cells are not dissociated from each other. At days 2 and 4, a 200 ml bolus of media supplemented with cytokines is supplied. The harvesting conditions and all other conditions are the same as in Example 1.

### Example 3: CFU Assays

Colony Forming Units or "CFUs" are a means to judge the stemness of cells in an in vitro model. A known number of cells are plated on a methylcellulose coated culture flask with a defined media (i.e. Methocult). After two weeks, colonies having defined morphologies are formed by presumptive stem cells. These colonies are quantifiable and can be used to give a determination of HPSCs within a given population. The expansion rates of the HSPCs was highest in the bioreactor, followed by the flask-based expansion with the E40RF 1+ ECs, and the cytokine alone expansions providing the least amount of HSPC expansion (see Figure 7).

### Example 4: Flow Cytometry

Hematopoietic cells, either from freshly isolated umbilical cord blood (un-manipulated), from cells expanded with cytokines alone for 6 days, from cells expanded on E4ORF1+ ECs in a flask for 6 days, or from cells expanded on E40RF 1+ ECs in a hollow fiber bioreactor for 6 days, were all analyzed via flow cytometry. The populations of cells were stained for CD34, CD45, and CD38, simultaneously. Each parameter was distinguishable via the unique fluorophores conjugated to the antibodies specific for those cell surface antigens. CD45- cells were identified as the E4ORF1+ engineered ECs. CD45 is a pan-hematopoietic marker. CD45+ CD34+ cells comprise a more primitive population of HSPCs within the total CD45+ population, compared to CD45+ CD34- cells. CD45+ CD34+ CD38- cells comprise an even more primitive HSPC population. The expansion rates of these primitive populations were consistently highest in the bioreactor, followed by the flask-based expansion with the E40RF 1+ engineered ECs, with the cytokine alone treatment providing the least amount of expansion.

### Example 4: Transplantation of Expanded HSPCs

HSPCs expanded by continuous contact with E4ORF1+ engineered ECs may produce a superior engraftable product, for example as compared to HSPCs obtained via expansions where the interactions are disrupted via serial passaging and re-plating of HSPCs. By eliminating this disruption, HSPCs expanded in this novel fashion may lead to more rapid engraftment of the lymphoid, myeloid, and erythroid lineages resulting in their respective blood counts returning faster after myeloablation. This should also improve long-term engraftment, as the use of E4ORF1+ engineered ECs for expansion is hypothesized to retain long term repopulating stem cells. Expansion by continuous contact with E4ORF1+ engineered ECs may also result in generation of sufficient HSPC material for one adult to receive a graft from a single UCB unit from a single donor, or even multiple transfusions of the graft into a single adult, or for multiple transfusions from a single UCB donor into multiple adults. The expected rapid engraftment may also result in a range of other benefits, including possibly reduced hospital stays, lower rates of GVHD of all grades, decreased levels of relapse, decreased transplant-related mortality (TRM), decreased risk of infection, reduced incidents of graft failure, the ability to use smaller donations of cord blood that would otherwise be unusable, increased access to UCB donations for cord blood of rare HLA type, an overall reduction in the cost of treatment, increased life-span for transplant recipients, and/or an increase in the quality of life of transplant recipients.

The foregoing descriptions of specific embodiments of the present invention have been presented for purposes of illustration and description. They are not intended to be exhaustive or to limit the invention to the precise forms disclosed, and obviously many modifications and variations are possible in light of the above teaching. The embodiments were chosen and described in order to best explain the principles of the invention and its practical application, to thereby enable others skilled in the art to best utilize the invention and various embodiments with various modifications as are suited to the particular use contemplated.

## Claims

1. A pharmaceutical composition comprising: (a) an expanded population of human umbilical cord blood hematopoietic stem and/or progenitor cells (HSPCs), (b) E4ORF1+ human umbilical vein endothelial cells (E4ORF1+ HUVECs), and (c) a pharmaceutically acceptable carrier, wherein the pharmaceutical composition is for use in stem cell transplantation in a human subject in need of HSPC transplantation.

2. The composition according to claim 1, wherein the umbilical cord blood HSPCs are hematopoietic stem cells.

3. The composition according to any one of claims 1 to 2, wherein the umbilical cord blood HSPCs are allogeneic with respect to the subject.

4. The composition according to any one of claims 1 to 2, wherein the umbilical cord blood HSPCs are autologous with respect to the subject.

5. The composition according to any one of claims 1 to 4, wherein the E4ORF1+ HUVECs are mitotically inactivated.

6. The composition according to any one of claims 1 to 5, wherein the E4ORF1+ HUVECs are ETV2+.

7. The composition according to claim any one of claims 1 to 6, wherein the E4ORF1+ HUVECs are E4ORF6⁺.

8. The composition according to any one of claims 1 to 7, wherein the subject has a deficiency in hematopoiesis caused by a myeloablative treatment, or a disorder selected from: a hematologic disease, a disorder that requires bone marrow hematopoietic stem cell transplantation, an infectious immunodeficiency, an infectious disease affecting T cells, HIV, a genetic immunodeficiency, severe combined immunodeficiency, a genetic disease affecting erythrocytes, an anemia, and Fanconi anemia.

## Patentansprüche

1. Pharmazeutische Zusammensetzung, umfassend: (a) eine expandierte Population von hämatopoetischen Stamm- und/oder Vorläuferzellen (HSPCs) aus menschlichem Nabelschnurblut, (b) E4ORF1+-Endothelzellen der menschlichen Nabelschnurvene (E40RF1+-HUVECs) und (c) einen pharmazeutisch verträglichen Träger, wobei die pharmazeutische Zusammensetzung zur Verwendung bei einer Stammzelltransplantation bei einem menschlichen Subjekt bestimmt ist, das eine HSPC-Transplantation benötigt.

2. Zusammensetzung nach Anspruch 1, wobei es sich bei den HSPCs aus Nabelschnurblut um hämatopoetische Stammzellen handelt.

3. Zusammensetzung nach einem der Ansprüche 1 bis 2, wobei die HSPCs aus Nabelschnurblut in Bezug auf das Subjekt allogen sind.

4. Zusammensetzung nach einem der Ansprüche 1 bis 2, wobei die HSPCs aus Nabelschnurblut in Bezug auf das Subjekt autolog sind.

5. Zusammensetzung nach einem der Ansprüche 1 bis 4, wobei die E4ORF1+-HUVECs mitotisch inaktiviert sind.

6. Zusammensetzung nach einem der Ansprüche 1 bis 5, wobei die E4ORF1+-HUVECs ETV2+ sind.

7. Zusammensetzung nach einem der Ansprüche 1 bis 6, wobei die E4ORF1+-HUVECs E4ORF6⁺ sind.

8. Zusammensetzung nach einem der Ansprüche 1 bis 7, wobei das Subjekt einen durch eine myeloablative Behandlung verursachten Mangel an Hämatopoese aufweist oder an einer der folgenden Störungen leidet, ausgewählt aus: einer hämatologischen Krankheit, einer Krankheit, die eine Transplantation von hämatopoetischen Stammzellen aus dem Knochenmark erfordert, einer infektiösen Immundefizienz, einer T-Zellen-befallenden Infektionskrankheit, HIV, einer genetischen Immundefizienz, einem schweren kombinierten Immundefekt, einer genetischen Krankheit, die Erythrozyten betrifft, einer Anämie und einer Fanconi-Anämie.

## Revendications

1. Composition pharmaceutique comprenant : (a) une population étendue de cellules souches et/ou progénitrices hématopoïétiques (HSPC) issues du sang de cordon ombilical humain, (b) des cellules endothéliales de veine ombilicale humaine E4ORF1+ (HUVEC E4ORF1+), et (c) un excipient pharmaceutiquement acceptable, dans laquelle la composition pharmaceutique est destinée à être utilisée dans une greffe de cellules souches chez un sujet humain ayant besoin d'une greffe HSPC.

2. Composition selon la revendication 1, dans laquelle les cellules HSPC issues du sang de cordon ombilical sont des cellules souches hématopoïétiques.

3. Composition selon l'une quelconque des revendications 1 à 2, dans laquelle les cellules HSPC issues du sang de cordon ombilical sont allogéniques par rapport au sujet.

4. Composition selon l'une quelconque des revendications 1 à 2, dans laquelle les cellules HSPC issues du sang de cordon ombilical sont autologues par rapport au sujet.

5. Composition selon l'une quelconque des revendications 1 à 4, dans laquelle les cellules HUVEC E4ORF1+ sont inactivées mitotiquement.

6. Composition selon l'une quelconque des revendications 1 à 5, dans laquelle les cellules HUVEC E4ORF1+ sont des cellules ETV2+.

7. Composition selon l'une quelconque des revendications 1 à 6, dans laquelle les cellules HUVEC E4ORF1+ sont des cellules E4ORF6⁺.

8. Composition selon l'une quelconque des revendications 1 à 7, dans laquelle le sujet présente un déficit hématopoïétique causé par un traitement myéloablatif, ou un trouble sélectionné parmi : une maladie hématologique, un trouble qui nécessite une greffe de cellules souches hématopoïétiques de moelle osseuse, une immunodéficience infectieuse, une maladie infectieuse affectant les cellules T, le VIH, une immunodéficience génétique, une immunodéficience combinée sévère, une maladie génétique affectant les érythrocytes, une anémie et l'anémie de Fanconi.
